Europäisches Patentamt

(19) European Patent Office

Office européen des brevets

(11) Publication number: **0 272 858**
**A2**

# EUROPEAN PATENT APPLICATION

(21) Application number: **87310967.2**

(22) Date of filing: **14.12.87**

(51) Int. Cl.⁴: **C 12 N 15/00**
**C 12 N 5/00, C 12 N 7/00,**
**C 12 N 1/20, G 01 N 33/569,**
**C 07 K 15/04, A 61 K 39/21,**
**C 07 H 21/04**

(30) Priority: **31.08.87 US 91481   15.12.86 US 941111**

(43) Date of publication of application:
**29.06.88 Bulletin 88/26**

(84) Designated Contracting States:
**AT BE CH DE ES FR GB GR IT LI LU NL SE**

(71) Applicant: **REPLIGEN CORPORATION**
**101 Binney Street**
**Cambridge Massachusetts 02142 (US)**

(72) Inventor: **Rusche, James**
**18 Brigham Road**
**Framingham Massachusetts 01701 (US)**

**Lynn, Debra**
**11 Allen Street Apt. 11**
**Arlington Massachusetts 02174 (US)**

**Carson, Helen**
**39 Sprague Street**
**Malden Massachusetts 02148 (US)**

**Putney, Scott**
**5 Epping Street**
**Arlington Massachusetts 02174 (US)**

**Jellis, Cindy Lou**
**48 Redfield Circle**
**Derry New Hampshire 03038 (US)**

(74) Representative: **Perry, Robert Edward et al**
**GILL JENNINGS & EVERY 53-64 Chancery Lane**
**London WC2A 1HN (GB)**

The applicant has filed a statement in accordance with Rule 28 (4) EPC (issue of a sample only to an expert). Accession number(s) of the deposit(s): NRRLB 18150, 18151, 18152, 18248.

(54) **Recombinant HIV envelope proteins produced in insect cells.**

(57) The preparation of recombinant HIV envelope protein gp160, and variants thereof, is accomplished for the first time by use of insect cells and recombinant DNA technology. This progress for preparing intact gp160 is not predictable to any degree from known prior art processes, which process the gp160 to gp120 and gp41. Also disclosed and claimed is the preparation of HIV envelope protein gp120, and HIV variants of gp160. gp160 and gp120 can be used as immunogens to give rise to HIV antibodies.

EP 0 272 858 A2

**Description**

RECOMBINANT HIV ENVELOPE PROTEINS PRODUCED IN INSECT CELLS

This invention relates to recombinant HIV envelope proteins produced in insect cells.

The cause of acquired immune deficiency syndrome (AIDS) has been variously identified as human T-cell lymphotropic virus (HTLV-III), lymphadenopathy-associated virus (LAV) or AIDS-associated retrovirus (ARV); see Popovic et al, Science (1984) 224 497-500. The currently-accepted designation is HIV (human immunodeficiency virus), and HIV is used herein.

Antibodies against HIV proteins in the sera of most AIDS and AIDS-related complex (ARC) patients, and in asymptomatic people infected with the virus (Sarngadharan et al, Science (1984) 224 506-508), have made possible the development of immunologically-based tests that detect antibodies to these antigens. These tests are used to limit the spread of HIV by blood transfusion by identifying blood samples of people infected with the virus. Diagnostic tests currently commercially-available use the proteins of inactivated virus as antigens.

In addition to allowing new approaches for diagnosis recombinant DNA holds great promise for the development of vaccines against both bacteria and viruses (Wilson, T. [1984] Bio/Technology 2:29-39). The most widely employed organisms to express recombinant vaccines have been E. coli, S. cerevisiae and cultured mammalian cells.

One of the most pressing issues to resolve is the feasibility of a vaccine to protect against HIV infection. Important proteins to consider in this regard are those associated with the viral envelope. These are derived from a precursor, gp160, that is proteolytically cleaved to generate the external envelope glycoprotein gp120 and the transmembrane envelope glycoprotein gp41. Several lines of evidence support the notion that the envelope protein is involved in important biological processes related to the virus life cycle and that a subunit vaccine based on these proteins may be possible.

(1) Antibodies that neutralize HIV infection have been found in sera of people with AIDS and ARC (AIDS related complex), as well as in sera of asymptomatic individuals infected with the virus. Antibodies exist in such sera that bind to purified gp120.

(2) Purified gp120 elicits production of neutralizing antibodies as does a large segment of gp120 produced in mammalian cells.

(3) Direct binding of gp120 to the T4 receptor has been demonstrated, and monoclonal antibodies recognizing certain epitopes of the virus receptor prevent viral infection.

Heretofore, gp160 has not been produced via recombinant means because expression of the gene coding for gp160 results in its proteolytic cleavage to gp120 and gp 41, as discussed above. Thus there exists no known prior art recombinant procedure for expressing gp160 and purifying this protein. Further, there is no known prior art which suggests that gp160 could be used as an immunogen to give rise to HIV neutralizing antibodies.

gp160 has not been purified in the prior art and has only been observed by polyacrylamide gel electrophoresis of virus and virus infected cells. As it exists on such gels, gp160 is impure because it co-migrates with contaminating, cellular proteins. Elution of the gp160 band from these gels would not result in pure protein. This disclosure provides a method to make recombinant gp160 in its essentially pure form. Such a preparation of gp160 is useful as a vaccine in contrast to unpurified gp160 as part of a whole virus or virus infected cells, because a much stronger immune response can be elicited by purified gp160. In addition, vaccination with virus is not desirable because of the possibility of viral infection.

The subject invention provides a means via recombinant DNA technology to produce large quantities of HIV envelope proteins gp160 and gp120. More specifically, gp160 and gp120 are produced in and purified from insect cells using recombinant DNA procedures. It was surprising that intact gp160 in insect cells would not be processed to gp120 and gp41 for the following reasons:

It is well known that (1) gp160 is processed in the mammalian cell lines HeLa, BSC-40, and H9 cells (Chakrabarti, S., Robert-Guroff, M. Wong-Staal, F., Gallo, R.C. and Moss, B. [1986] Nature 320:535-537; Hu, S.L., Kosowski, S.G. and Dalyrymple, J.M. [1986] Nature 320:537-540) and (2) insect cells are capable of correctly processing the secretion signal from other mammalian genes such as IL-2 (Smith, G.E., Ju, G., Ericson, B.L., Moschera, J., Lahm, H.W., Chizzonite, R. and Summers, M.D. [1985] Proc. Natl. Acad. Sci. USA 82:8404-8408). Therefore, one would expect that they would process gp160.

This unexpected result of obtaining intact gp160 from insect cells makes possible the production of large amounts of gp160 for testing and use as an immunogen to give rise to HIV antibodies. gp160 has never been tested for such use due to the difficulty in producing it. The subject invention is the first known solution to this problem.

Upon producing gp160 via the invention process, it was found, again quite unexpectedly and advantageously, that gp160 gives humoral and cellular immunity in goats immunized with purified gp160. Most advantageously, HIV neutralizing antibody titers elicited by gp160 produced in insect cells were found to be 5-fold higher than antibody titers elicited by native gp120. The neutralizing titers elicited by gp160 are also several-fold higher than neutralizing antibody titers from human AIDS and ARC patients.

This is the first known demonstration of an HIV recombinant envelope protein that gives both humoral and cellular immunity.

The HIV envelope protein gp120 has been purified from virus-infected cells and elicits neutralizing antibodies and cellular immunity (Robey, W.V., Arthur, L.O., Matthews, T.J., Langlois, A., Copeland, T.D., Lerche, N.W., Oroszlan, S., Bolognesi, D.P., Gilden, R.V. and Fischinger, P.J. [1986] Proc. Natl. Acad. Sci. USA 83:7023-7027). Recombinant gp160, described herein, elicits neutralizing antibody titers 5-fold higher than gp120. By expressing gp120 in insect cells, the protein may have essentially the same increased antigenic properties in eliciting neutralizing antibodies as gp160 from insect cells. gp120 can be expressed in insect cells by introducing a termination codon at the end of the gp120 gene (amino acid number 511) in the recombinant baculovirus clone HT3.

Envelope proteins gp160 and gp120 can be used as a vaccine, diagnostic and therapy for AIDS. gp160 elicits antibodies capable of preventing HIV mediated cell fusion of HIV-infected cells. Further, gp160 can be used to stimulate a lymphocyte proliferative response in HIV-infected humans. This then would stimulate the immune system to respond to HIV in such individuals and, therefore, gp160 can provide protection and be of therapeutic value. gp120 expressed in insect cells is immunoreactive to antisera raised to gp160 and should have essentially the same anti-HIV immunological properties as gp160 from insect cells.

The invention also concerns gp160 and gp120 variants. These variants are constructed for expression in insect cells. The proteins produced are useful in the same maner as described herein for the parent proteins.

The following deposits have been made in the Agricultural Research Service Patent Culture Collection (NRRL), Northern Regional Research Center, 1815 North University Street, Peoria, Illinois 60604, USA.

| Culture | Deposit No. | Deposit Date |
|---------|-------------|--------------|
| E. coli JM103(pSP64) | NRRL B-18150 | 04.12.86 |
| E. coli HM101(pAcHT3) | NRRL B-18152 | 04.12.86 |
| E. coli JM103(pAc610) | NRRL B-18151 | 04.12.86 |
| E. coli JM103(pAcHT7) | NRRL B-18248 | 07.08.87 |

The insect cell line Sf9 (Spodoptera frugiperda), deposit number ATCC CRL 1711, is available from the American Type Culture Collection, 12301 Parklawn Drive, Rockville, Maryland 20852, USA. Baculovirus Autrographa californica nuclear polyhedrosis virus (AcNPV) is available from Texas A & M University, Texas Agricultural Experiment Station, College Station, Texas 77843, USA, and has been described in Smith et al, (1978) Virology 89 517-527 and (1979) J. Virology 30 828-838.

The restriction enzymes disclosed can be purchased from Bethesda Research laboratories, Gaithersburg, Maryland, USA, or New England Biolabs, Beverly, Massachusetts, USA. The enzymes are used according to the instructions provided by the supplier.

The various methods employed in the preparation of the plasmids and transformation of host organisms are well known in the art. These procedures are all described by Maniatis et al (1982) Molecular Cloning: A Laboratory Manual, Cold Spring Harbor Laboratory, New York, USA. Thus, it is within the skill of those in the genetic engineering art to extract DNA from microbial cells, perform restriction enzyme digestions, electrophorese DNA fragments, tail and anneal plasmid and insert DNA, ligate DNA, transform cells, e.g., E. coli cells, prepare plasmid DNA, electrophorese proteins, and sequence DNA.

Other nuclear polyhedrosis viruses (See World Health Organization Technical Report No. 531) such as Spodoptera frugiperda (Sf MNPV), Choristoneura fumiferana (Cf MNPV) (Smith, G. and Summers, M.D. [1981] J. Virol. 39:125-137), or Spodoptera littoralis (Sl NPV) (Harrap, K.A., Payne, C.C. and Robertson, J.S. [1977] Virology 79:14-31) can be used instead of Autographa californica NPV. Other insect cell lines can also be substituted for Spodoptera frugiperda (Sf9), for example, Trichoplusia ni (Volkman, L.E. and Summers, M.D. [1975] J. Virol. 16:1630-1637), Spodoptera exigua, Choristoneura fumiferana (Smith, G. and Summers, M.D. [1981] J. Virol. 39:125-137) and Spodoptera littoralis (Harrap, K.A. et al. [1977] Virology 79:14-31).

The accompanying drawing shows the construction of pAcHT3. In the drawing, Lig = ligate, Tr = transform, and Syn olig = synthetic oligonucleotide.

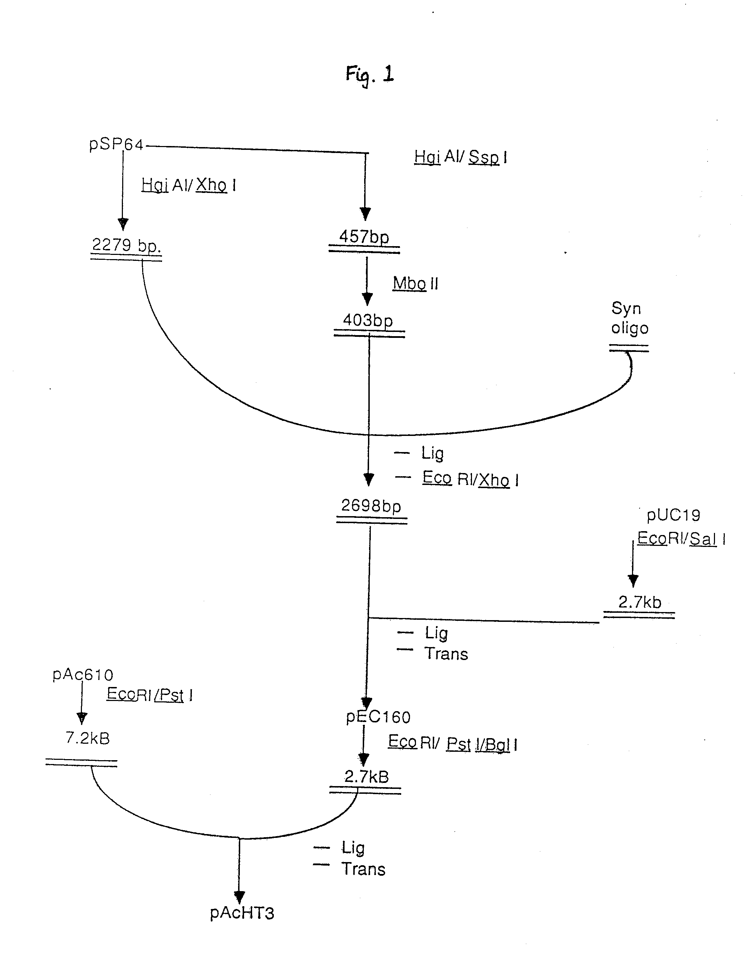

Using the Baculovirus expression vector, we have expressed the HIV envelope primary translation product, gp160. It is expressed using the polyhedrin gene promotor and 4' untranslated region and the HIV envelope secretion signal sequence (Figure 1). The protein product migrates on denaturing polyacrylamide gels as a 160 kD protein. That the protein is extensively glycosylated is demonstrated by staining gp160 with the carbohydrate-specific stain perchloric acid. After treatment with an endoglycosidase, for example, N-GLYCANASE™, (Genzyme Corp., Boston MA), the protein migrates at 96 kD on denaturing polyacrylamide gels, only slightly larger than the size predicted for the entire envelope polypeptide. The recombinant envelope protein has been localized to the cell surface of infected insect cells, indicating secretion and insertion in the cell membrane.

To obtain immune sera to gp160, goats were immunized with pure gp160 or whole insect cells infected with the HIV recombinant baculovirus. The adjuvant was complete Freunds adjuvant. Pure gp160 protein for immunization was obtained from preparative denaturing polyacrylamide gels. Sera from goats immunized with pure gp160 or insect cells expressing gp160 specifically bind gp160, gp120, and gp41 on Western blots. Therefore, animals immunized with recombinant gp160 develop immune recognition to the natural viral envelope of HIV.

Immunochemical assays employing the HIV proteins of the invention can take a variety of forms. The preferred type is a solid phase immunometric assay. In assays of this type, an HIV protein is immobilized on a solid phase to form an antigen-immunoadsorbent. The immunoadsorbent is incubated with the sample to be tested. After an appropriate incubation period, the immunoadsorbent is separated from the sample and labeled anti-(human IgG) antibody is used to detect human anti-HIV antibody bound to the immunoadsorbent. The amount of label associated with the immunoadsorbent can be compared to positive and negative controls to assess the present or absence of anti-HIV antibody.

The immunoadsorbent can be prepared by adsorbing or coupling a purified HIV protein to a solid phase. Various solid phases can be used, such as beads formed of glass, polystyrene, polypropylene, dextran or other material. Other suitable solid phases include tubes or plates formed from or coated with these materials.

The HIV proteins can be either covalently or non-covalently bound to the solid phase by techniques such as covalent bonding via an amide or ester linkage or by adsorption. After the HIV protein is affixed to the solid phase, the solid phase can be post-coated with an animal protein, e.g., 3% fish gelatin. This provides a blocking protein which reduces nonspecific adsorption of protein to the immunoadsorbent surface.

The immunoadsorbent is then incubated with the sample to be tested for anti-HIV antibody. In blood screening, blood plasma or serum is used. The plasma or serum is diluted with normal animal plasma or serum. The diluent plasma or serum is derived from the same animal species that is the source of the anti-(human IgG) antibody. The preferred a anti-(human IgG) antibody is goat anti-(human IgG) antibody. Thus, in the preferred format, the diluent would be goat serum or plasma.

The conditions of incubation, e.g., pH and temperature, and the duration of incubation are not crucial. These parameters can be optimized by routine experimentation. Generally, the incubation will be run for 1-2 hr at about 45°C in a buffer of pH 7-8.

After incubation, the immunoadsorbent and the sample are separated. Separation can be accomplished by any conventional separation technique such as sedimentation or centrifugation. The immunoadsorbent then may be washed free of sample to eliminate any interfering substances.

The immunoadsorbent is incubated with the labeled anti-(human IgG) antibody (tracer) to detect human antibody bound thereto. Generally the immunoadsorbent is incubated with a solution of the labeled anti-(human IgG) antibody which contains a small amount (about 1%) of the serum or plasma of the animal species which serves as the source of the anti-(human IgG) antibody. Anti-(human IgG) antibody can be obtained from any animal source. However, goat anti-(human IgG) antibody is preferred. The anti-(human IgG) antibody can be an antibody against the Fc fragment of human IgG, for example, goat anti-(human IgG) Fc antibody.

The anti-(human IgG) antibody or anti-(human IgG)Fc can be labeled with a radioactive material such as $^{125}$iodine; labeled with an optical label, such as a fluorescent material; or labeled with an enzyme such as horseradish peroxidase. The anti-human antibody can also be biotinylated and labeled avidin used to detect its binding to the immunoadsorbent.

After incubation with the labeled antibody, the immunoadsorbent is separated from the solution and the label associated with the immunoadsorbent is evaluated. Depending upon the choice of label, the evaluation can be done in a variety of ways. The label may be detected by a gamma counter if the label is a radioactive gamma emitter, or by a fluorimeter, if the label is a fluorescent material. In the case of an enzyme, label detection may be done colorimetrically employing a substrate for the enzyme.

The amount of label associated with the immunoadsorbent is compared with positive and negative controls in order to determine the presence of anti-HIV antibody. The controls are generally run concomitantly with the sample to be tested. A positive control is a serum containing antibody against HIV; a negative control is a serum from healthy individuals which does not contain antibody against HIV.

For convenience and standardization, reagents for the performance of the immunometric assay can be assembled in assay kits. A kit for screening blood for example, can include:

    (a) An immunoadsorbent, e.g., a polystyrene bead coated with an HIV protein;

    (b) a diluent for the serum or plasma sample, e.g., normal goat serum or plasma;

    (c) an anti-(human IgG) antibody, e.g., goat anti-(human IgG) antibody in buffered, aqueous solution containing about 1% goat serum or plasma;

    (d) A positive control, e.g., serum containing antibody against at least one of the novel HIV proteins; and

    (e) a negative control, e.g., pooled sera from healthy individuals which does not contain antibody against at least one of the novel HIV proteins.

If the label is an enzyme, an additional element of the kit can be the substrate for the enzyme.

Another type of assay for anti-HIV antibody is an antigen sandwich assay. In this assay, a labeled HIV protein is used in place of anti-(human IgG) anti body to detect anti-HIV antibody bound to the immunoadsorbent. The assay is based in principle on the bivalency of antibody molecules. One binding site of the antibody binds the antigen affixed to the solid phase; the second is available for binding the labeled antigen. The assay procedure is essentially the same as described for the immunometric assay except that after incubation with the sample, the immunoadsorbent is incubated with a solution of labeled HIV protein. HIV proteins can be labeled with radioisotope, an enzyme, etc. for this type of essay.

In a third format, the bacterial protein, protein A, which binds the Fc segment of an IgG molecule without interfering with the antigen-antibody interaction can be used as the labeled tracer to detect anti-HIV antibody adsorbed to the immunoadsorbent. Protein A can be readily labeled with a radioisotope, enzyme or other detectable species.

Immunochemical assays employing an HIV protein have several advantages over those employing a whole (or disrupted) virus. Assays based upon an HIV protein will alleviate the concern over growing large quantities of infectious virus and the inherent variability associated with cell culturing and virus production. Further, the assay will help mitigate the real or perceived fear of contracting AIDS by technicians in hospitals, clinics and blood banks who perform the test.

Vaccines comprising one or more of the HIV proteins, disclosed herein, and variants thereof having antigenic properties, can be prepared by procedures well known in the art. For example, such vaccines can be prepared as injectables, e.g., liquid solutions or suspensions. Solid forms for solution in, or suspension in, a liquid prior to injection also can be prepared. Optionally, the preparation also can be emulsified. The active antigenic ingredient or ingredients can be mixed with excipients which are pharmaceutically acceptable and compatible with the active ingredient. Examples of suitable excipients are water, saline, dextrose, glycerol, ethanol, or the like, and combinations thereof. In addition, if desired, the vaccine can contain minor amounts of auxiliary substances such as wetting or emulsifying agents, pH buffering agents, or adjuvants such as aluminum hydroxide or muramyl dipeptide which enhance the effectiveness of the vaccine. Such adjuvants include aluminum hydroxide, threonyl-muramyl dipeptide (Allison, A.C. et al., Immunologic Adjuvants: Efficacy and Safety Conditions, in R.J. Nervig et al. eds. Adv. in Carriers and Adjuvants for Vet. Biologics, Iowa State U. Press [1986], or immune stimulatory complexes (ISCOMS) (Morein, B. et al. [1984] Nature 308:457-460). The vaccines are conventionally administered parenterally, by injection, for example, either subcutaneously or intramuscularly. Additional formulations which are suitable for other modes of administration include suppositories and, in some cases, oral formulations. For suppositories, traditional binders and carriers include, for example, polyalkalene glycols or triglycerides. Suppositories can be formed from mixtures containing the active ingredient in the range of about 0.5% to about 10%, preferably about 1 to about 2%. Oral formulations can include such normally employed excipients as, for example, pharmaceutical grades of manitol, lactose, starch, magnesium stearate, sodium saccharine, cellulose, magnesium carbonate and the like. These compositions can take the form of solution, suspensions, tablets, pills, capsules, sustained release formulations or powders and contain from about 10% to about 95% of active ingredients, preferably from about 25% to about 70%.

The proteins can be formulated into the vaccine as neutral or salt forms. Pharmaceutically acceptable salts include the acid addition salts (formed with the free amino groups of the peptide) and which are formed with inorganic acids such as, for exmaple, hydrochloric or phosphoric acids, or such organic acids as acetic, oxalic, tartaric, mandelic and the like. Salts formed with the free carboxyl groups also can be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

The vaccines are administered in a manner compatible with the dosage formulation, and in such amount as will be therapeutically effective an immunogenic. The quantity to be administered depends on the subject to be treated, capacity of the subject's immune system to synthesize antibodies, and the degree of protection desired. Precise amounts of active ingredient required to be administered depend on the judgment of the practitioner and are peculiar to each individual. However, suitable dosage ranges are of the order of about several hundred micrograms active ingredient per individual. Suitable regimes for initial administration and booster shots are also variable, but are typified by an initial administration followed in oneor two-week intervals by a subsequent injection or other administration.

HIV is known to undergo amino acid sequence variation, particularly in the envelope gene (Starcich, B.R. [1986] Cell 45:637-648; Hahn, B.H. et al. [1986] Science 232:1548-1553). Over 100 variants have been analyzed by molecular cloning and restriction enzyme recognition analysis, and several of these have been analyzed by nucleotide sequencing. Some of these are the HIV isolates known as RF (Popovic, M. et al. [1984] Science 224:497-500, WMJ-1 (Hahn, B.H. et al. [1986] Science 232:1548-1553, LAV (Wain-Hobson, S. et al. [1985] Cell 40:9-17, and ARV-2 (Sanchez-Pescador, R. et al. [1985] Science 227:484-492). Although the subject invention describes one HIV isolate, two variants, and a hybrid polypeptide between two variants, the appropriate envelope regions of any HIV isolate can be produced using the procedures described herein. The HIV proteins from different viral isolates can be used in vaccine preparations, as disclosed above, to protect against infections by different HIV isolates. Further, a vaccine preparation can be made using more than one HIV isolate to provide immunity and thus give better protection against AIDS.

The HIV proteins of the subject invention can be chemically synthesized by solid phase peptide synthetic techniques such as BOC and FMOC (Merrifield, R.B. [1963] J. Amer. Chem. Soc. 85:2149; Chang, C. and Meienhofer, J. [1978] Int. J. Peptide Protein Res. 11:246).

As is well known in the art, the amino acid sequence of a protein is determined by the nucelotide sequence of the DNA. Because of the redundancy of the genetic code, i.e., more than one coding nucleotide triplet (codon) can be used for most of the amino acids used to make proteins, different nucleotide sequences can code for a particular amino acid. Thus, the genetic code can be depicted as follows: Phenylalanine (Phe)    TTK

5

Leucine (Leu)    XTY
Isoleucine (Ile)    ATM
Methionine (Met)    ATG
Valine (Val)    GTL
Serine (Ser)    QRS
Proline (Pro)    CCL
Threonine (Thr)    ACL
Alanine (Ala)    GCL
Tyrosine (Tyr)    TAK
Termination Signal    TAJ
Termination Signal    TGA
Histidine (His)    CAK
Glutamine (Gln)    CAJ
Asparagine (Asn)    AAK
Lysine (Lys)    AAJ
Aspartic acid (Asp)    GAK
Glutamic acid (Glu)    CAJ
Cysteine (Cys)    TGK
Tryptophan (Trp)    TGG
Arginine (Arg)    WGZ
Glycine (Gly)    GGL

Key: Each 3-letter deoxynucleotide triplet corresponds to a trinucleotide of mRNA, having a 5'-end on the left and a 3'-end on the right. All DNA sequences given herein are those of the strand whose sequence corresponds to the mRNA sequence, with thymine substituted for uracil. The letters stand for the purine or pyrimidine bases forming the deoxynucleotide sequence.

A = adenine
G = guanine
C = cytosine
T - thymine
X = T or C if Y is A or G
X = C if Y is C or T
Y = A, G, C or T if X is C
Y = A or G if X is T
W = C or A if Z is A or G
W = C if Z is C or T
Z = A, G, C or T if W is C
Z = A or G if W is A
QR = TC if S is A, G, C or T; alternatively QR = AG if S is T or C
J = A or G
K = T or C
L = A, T, C or G
M = A, C or T

The above shows that the novel amino acid sequences of the HIV proteins of the subject invention can be prepared by nucleotide sequences other than those disclosed herein. Functionally equivalent nucleotide sequences encoding the novel amino acid sequences of these HIV proteins, or fragments thereof having HIV antigenic or immunogenic or therapeutic activity, can be prepared by known synthetic procedures. Accordingly, the subject invention includes such functionally equivalent nucleotide sequences.

Thus the scope of the subject invention includes not only the specific nucleotide sequences depicted herein, but also all equivalent nucleotide sequences coding for molecules with substantially the same HIV antigenic or immunogenic or therapeutic activity.

Further, the scope of the subject invention is intended to cover not only the specific amino acid sequences disclosed, but also similar sequences coding for proteins or protein fragments having comparable ability to induce the formation of and/or bind to specific HIV antibodies.

The term "equivalent" is being used in its ordinary patent usage here as denoting a nucleotide sequence which performs substantially as the nucleotide sequence identified herein to produce molecules with substantially the same HIV antigenic or immunogenic or therapeutic activity in essentially the same kind of hosts. Within this definition are subfragments which have HIV antigenic or immunogenic or therapeutic activity.

As disclosed above, it is well within the skill of those in the genetic engineering art to use the nucleotide sequences encoding HIV antigenic or immunogenic or therapeutic activity of the subject invention to produce HIV proteins via microbial processes. Fusing the sequences into an expression vector and transforming or transfecting into hosts, either eukaryotic (yeast or mammalian cells) or prokaryotic (bacterial cells), are standard procedures used in producing other well-known proteins, e.g., insulin, interferons, human growth hormone, IL-1, IL-2, and the like. Similar procedures, or obvious modifications thereof, can be employed to prepare HIV proteins by microbial means or tissue-culture technology in accord with the subject invention.

The nucleotide sequences disclosed herein can be prepared by a "gene machine" by procedures well known in the art. This is possible because of the disclosure of the nucleotide sequence.

Following are examples which illustrate the invention, including the best mode. These examples should not be construed as limiting. All solvent mixture proportions are by volume unless otherwise noted.

The disclosure of the production of gp160 from insect cells includes five parts:

(1) construction of the gp160 encoding plasmid pAcHT3;
(2) transfection of this plasmid into Sf9 (Spodoptera frugiperda) insect cells;
(3) isolation of recombinant Baculovirus;
(4) infection and growth of infected cells; and
(5) purification of recombinant gp160.

Example 1--Plasmid construction

The construction of the gp160 encoding plasmid is diagramed In Figure 1. The starting plasmid of this construction is pSP64, which has been deposited in the ARS Patent Culture Collection, Northern Regional Research Center, 1815 University Street, Peoria, Illinois 61604 USA. The accession number assigned to the deposit by the repository is NRRL B-18150 and the deposit date is December 4, 1986. The construction was done as follows:

1. The DNA fragment encoding the carboxyl-terminal segment of gp160 was isolated by digesting pSP64 with HgiAl and XhoI. The resulting 2279 base pair (bp) fragment which extends from within the envelope gene to beyond the translation termination codon of gp160, was isolated from an agarose gel by standard procedures. Alternatively, this gene can be synthesized. The sequence is listed in the following references: Ratner, L., et al. (1985) Nature 313: 277-284; Starcich, B.R. (1986) Cell 45:637-648; Hahn, B.H., et al. (1986) Science 232:1548-1553; Wain-Hobson, S., et al. (1985) Cell 40:9-17; and Sanchez-Pescador, R. et al. (1985) Science 227:484-492.

2. The DNA encoding the amino terminal region of gp160 was isolated by digesting pSP64 with HgiAl and SspI and purifying the resulting 457 bp fragment. This fragment was then digested with MboII, which cleaves immediately upstream from the envelope start codon. The 403 bp fragment was gel purified by standard procedures.

3. The following synthetic double-stranded oligonucleotide was constructed by synthesizing each strand indpendently and purifying them from a 12% polyacrylamide gel. The strands were annealed by heating to 100°C and rapid cooling on ice water (Rossi, et al. [1982] J. Biol. Chem. 257:9226-9229):

```
5'    AATTCAACCTATAAATA    3'

3'          GTTGGATATTTA    5'
```

4. Quantities of the 3 double-stranded DNA fragments obtained from procedures 1-3 above were combined and ligated using T4 DNA ligase. The mixture was then digested with both EcoRI and XhoI, and the resulting 2698 bp fragment was gel purified by standard procedures.

5. Plasmid pUC19 (New England Biolabs, Beverly, MA) was digested with EcoRI and SalI and the large 2.7 kB fragment was gel purified, as above. Any E. coli plasmid cloning vector containing EcoRI and SalI sites, such as pBR322, can be used.

6. The fragments from 4 and 5 above were ligated and the mixture used to transform E. coli JM103. Other E. coli hosts available from New England Biolabs can be used in place of E. coli JM103. Ampicillin resistant colonies were selected and plasmid DNA was isolated by standard procedures, and the appropriate restriction pattern confirmed the insertion of the 2698 bp fragment.

7. This plasmid (pEc160) was then digested with a combination of EcoRI, PstI and BglI and the resultant 2.7 kB fragment was gel isolated, by standard procedures.

8. Plasmid pAc610 (NRRL B-18151) was digested with EcoRI and PstI and the 7,200 bp fragment gel isolated, as above.

9. The fragments resulting from procedures 7 and 8 above were ligated and used to transform competent cells of E. coli JM103. Other E. coli hosts can be used.

10. Restriction digests were performed to confirm the identity of this plasmid, designated pAcHT3, and several hundred micrograms of this plasmid were purified using the known cesium chloride gradient centrifugation.

Example 2--Transfection of Sf9 insect cells with pAcHT3

The following procedures involve use of the insect cell strain Sf9 and methods using the Baculovirus Autographa californica nuclear polyhedrosis virus (AcNPV). The standard protocols for growing and working with this cell line and this virus are described in A Manual of Methods for Baculovirus Vectors and Insect Culture Procedures, M.D. Summers and G.E. Smith, Texas Agricultural Experiment Station Bull. No. 1555, College Station, TX, 1986.

1. Sf9 cells were seeded into 25 cm² flasks at a density of 2.5 x 10⁶ cells per flask and allowed to attach to the flask surface. HEBS/CT and CaCl₂ solutions were warmed to room temperature at this time. 100 ml of a 10X stock solution of HEBS/CT had been prepared previously as follows:

```
1.37 M NaCl                      8.0   g
0.06 M D(+) glucose              1.081 g
0.05 M KCl                       0.373 g
0.007 M Na₂HPO₄·7H₂O             1.188 g
0.2 M HEPES (4-(2-               4.766 g
    hydroxyethyl-1-
    piperazine ethane-
    sulfonic acid)
```

The CaCl₂ solution was 2.5 M.

2. Medium was removed from flasks and replaced with 2 ml of Grace's Antheraea medium (Gibco Laboratories, Grand Island, NY) plus 10% fetal bovine serum (FBS) and antibiotics (50 μg/ml gentamycin and 2.5 μg/ml amphotericin B [FUNGIZONE, E.R Squibb and Sons, Princeton, NJ]). These flasks were then stored at room temperature.

3. 1 μg of AcNPV-E2 DNA and 2 μg of pAcHT3 recombinant plasmid DNA were mixed in a tube.

4. 950 μl of 1X HEBS/CT solution were added to the DNA tube and vortexed.

5. 50 μl of 2.5 M CaCl₂ were added to the DNA/HEBS/CT mixture, vortexed again, and then incubated at room temperature for 30 min.

6. The DNA/HEBS/CT solution was added to the Grace's medium already in the cell culture flasks.

7. Flasks were incubated at 27°C for 4 hr.

8. The medium was then removed from the flasks with a Pasteur pipet. After rinsing the flasks carefully with fresh Grace's medium plus 10% FBS plus antibiotics, 5 ml of TNM-FH (Grace's medium plus TC Yeastolate [Difco, Detroit, MI] at 13.32 g/4 liters and lactalbumin hydrolysate [Difco] at 13.32 g/4 liters) plus 10% FBS plus antibiotics were added.

9. The medium was then incubated at 27°C for 4-6 days; cells were periodically checked for signs of infection with an inverted microscope.

Example 3--Isolation and identification of recombinant viruses encoding gp160 by DNA hybridization

1. A flat bottom 96-well microtiter plate was seeded with 100 μl of Sf9 cells at a density of $1.5 \times 10^5$ cells/ml ($1.5 \times 10^4$ cells per well).

2. The sf9 cells were incubated at 27°C for 2-3 days under humidified conditions.

3. The medium was removed to a duplicate 96-well plate and stored at 4°C or frozen at -20°C.

4. Cells were lysed in the well by adding 200 μl of 0.5 N NaOH and mixing.

5. The solution was neutralized by adding 20 μl of 10 M NH₄Acetate and mixing.

6. The lysates were then spotted onto nitrocellulose filters in a dot blot (Bio-Rad Laboratories, Richmond, CA) as follows:

(a) A piece of nitrocellulose and Whatman 3MM paper (Whatman Laboratory Products, Clifton, NJ) were cut to size, the nitrocellulose was wet in hot water, and both were equilibrated in 1 M NH₄Acetate, 0.02 M NaOH.

(b) The blotting apparatus was set up as described by its manufacturer. The nitrocellulose was supported from below by the Whatman filter paper.

(c) The wells were rinsed with 1 M NH₄Acetate. All wells must filter solutions.

(d) Lysates were applied to the manifold.

(e) The wells were rinsed with 1 M NH₄Acetate.

(f) The nitrocellulose was removed and washed briefly (2 min) in 4X SSC (1X SSC = 0.15 M NaCl, 0.015 M NaCitrate, pH 7.0).

7. The filter was air dried and baked for 2 hr at 80°C under vacuum.

8. The material was hybridized with a $^{32}P$ labeled DNA fragment from the HgiAl to the XhoI site in the gene encoding gp160.

9. Viral candidates which contained the gp160 gene were identified by standard procedures.

10. To insure a pure clone the above recombinant virus identification scheme was repeated using the supernatant of the original positive well as starting material and virus was applied to wells at $10^7$, and $10^6$ dilutions.

11. The above step is repeated once again if necessary to obtain a pure gp160 encoding virus. This virus was denoted HT3. The titer of the virus was determined as described in Smith and Summers, supra.

## Example 4--Infection and growth of cultured insect cells with HT3

1. Cells were counted and seeded into flasks or plates containing medium TNM-FH plus 10% FBS plus antibiotics at an appropriate density and then allowed to attach to the flasks or plates for 30 min to 1 hr.

2. After cells were attached, the medium was removed and virus added at a multiplicity of infection (MOI) of 3.

3. Cells were incubated at 27° C for 1 hr.

4. Inoculum was removed and rinsed with the above medium (optional) and fresh complete medium (TNM-FH plus 10% FBS plus antibiotics) was added.

5. Cultures were incubated at 27° C for 2-4 days and examined daily.

6. The cell pellet was used for purification of gp160.

## Example 5--Recovery and purification of recombinant gp160 to its essentially pure form

1. 300 ml of cells in culture medium was centrifuged (3,000 xg, 10'), washed once with 20 ml phosphate buffered saline and the cells collected by centrifugation.

2. Cells were resuspended in 20 ml CMST buffer (0.03 M Tris pH 7.5, 0.01 M MgAcetate, 1% NONIDET P-40 [a non-ionic surfactant from Shell, Houston TX]) by vortexing and pipeting.

3. Cells wre pelleted for 5 min at 3,000 rpm.

4. Cells were resuspended in 20 ml 8 M urea buffer by vortexing for 5 min (8 M urea, 20 mM Tris pH 7.5, 15 mM 2-mercaptoethanol [BME], 1 mM ethylenediaminetetraacetic acid [EDTA], 1 mM phenylmethylsulfonyl fluoride [PMSF]).

5. Cells were centrifuged for 5 min at 8,000 rpm.

6. Supernatant was decanted and the pellet resuspended in 4 ml 0.1% sodium dodecylsulfate (SDS).

7. An equal volume of 2X SDS loading buffer was added, boiled for 5 min, and run on 7.5% SDS-polyacrylamide gel (18 x 16 x 0.3 mm) (75 mA for approximately 5-6 hr or 20 mA overnight).

8. To visualize gp160 protein:

   a. Gel was placed on a plate in ice cold 0.25 M KCl for 15 min (in refrigerator or freezer if possible).

   b. Gel was removed, wrapped with SARAN wrap (Dow Chemical, Midlan, MI) and left overnight at 4°C.

   c. The protein bands appeared white, and gp160 migrated at approximately 160 kD.

9. The gp160 band was cut from the gel.

10. gp160 was eluted from the gel by soaking the gel overnight in 20 mM Tris, pH 7.5, 0.1 M NaCl, and 0.1% SDS to give essentially pure gp160 protein.

## Example 6--Clone pAcHT7, gp160 of HIV variant RF

The cloning of the gp160 from variant RF (Popovic, M. et al. (1984) Science 224:497-500) was facilitated by using the plasmid (pEC160) described in Example 1, No. 7 as a starting point. This plasmid already has the necessary linkage sites for subcloning into the Baculovirus expression vector pAc610 as described in Example 1, Nos. 8-10. To insert gp160 sequences from RF, unique restriction enzyme sites within the two genes were used as described below:

Plasmid PEc160 was digested with DraIII and AvaI and the larger of the two fragments, 3600 bp, was isolated from an agarose gel by standard procedures. This fragment contains all of the pUC19 sequences as well as N-terminal amino acids of gp160 up to the DraIII site and C-terminal amino acids beyond the AvaI site. The major portion of the gp160 gene was provided by digestion of genomic clone of HIV variant RF. An 1828-bp fragment from RF envelope gene was prepared by DraIII/AvaI digestion and standard agarose gel techniques. This fragment contains the major portion of the envelope gene and has 88% of the variant amino acids of RF.

These fragments were ligated together and the mixture was used to transform E. coli JM103. Ampicillin-resistant colonies were selected and plasmid DNA was isolated by standard procedures.

The insertion of the 1828-bp fragment was confirmed by digestion with appropriate restriction enzymes. This plasmid, pHT7, was then digested as described in Example 1, No. 7 and cloned into pAc610 as described in Example 1, Nos. 8, 9, and 10. This plasmid is designated as pAcHT7. The amino acid sequence of the HIV protein expressed by this plasmid is shown in Table 1. The DNA sequence encoding this protein is shown in Table 2.

## Example 7--Clone pAcHT6, hybrid gp160 from HIV variants BH10 and RF

The cloning of a hybrid envelope gene from HIV variants BH10 and RF was accomplished as follows:

Plasmid pEc160 was used as a starting clone to provide the necessary linkages for subsequent subcloning into Baculovirus vector pAc610 as well as envelope sequences from HIV variant BH10. pEc160 has two BglII sites, both of which are within the gp160 gene. The larger fragment, 4800 bp, containing all of pUc19 as well as the first and last third of the total coding sequence of the gp160 gene was isolated from agarose gel by standard techniques.

The envelope subclone of the RF genomic clone was digested with BglII as well and a 565-bp fragment from the center of the envelope gene was isolated by standard techniques. This fragment was ligated to the pEc160 BglII 4800-bp fragment described above and the mixture was used to transform E. coli JM103. Ampicillin-resistant colonies were selected and plasmid DNA was isolated by standard procedures. pAcHT7 may be used as a source of RF sequences.

The insertion of the 565-bp fragment in the correct orientation was verified by digestion with appropriate restriction enzymes. This plasmid, pHT6, was then digested as described in Example 1, No. 7 and cloned into pAc610 as described in Example 1, Nos. 8, 9, and 10. This plasmid is designated pAcHT6 and is appropriate for expressing hybrid gp160 in insect cells. The amino acid sequence of the HIV protein expressed by this plasmid is shown in Table 3. The DNA sequence encoding this protein is shown in Table 4.

Transfection of Sf9 insect cells with pAcHT6 and pAcHT7 was done as described for pAcHT3. Subsequent steps involved in isolating the recombinant viruses and in infecting and growing cultured insect cells were also as described for pAcHT3.

Characteristics of variants

gp160 produced from clones pAcHT6 and pAcHT7 is similar to gp160 from pAcHT3 in its molecular weight on an SDS PAGE, and the amount of these proteins produced by insect cells is equivalent in all three cases. What distinguishes HT6 and HT7 from HT3, as well as from each other, are their immunological and antigenic characteristics. gp160 clones from variants present different epitopes which can be used in diagnostics as well as in vaccine development. Chimeric proteins may create unique epitopes that can be used in both these applications.

Immunology of HT6 and HT7

The gp160 produced from pAcHT6 is unique in that the central portion of the envelope is from HIV variant RF and the rest is from a distantly-related variant BH10. This hybrid protein has been shown to be immunogenic in goats and the antisera raised against HT6 has been analyzed by virus neutralization assays and cell-fusion inhibition assays. Results show that this hybrid protein is effective in these assays for both RF and BH10 variants.

Example 8--Construction of recombinant gp120 for expression in insect cells

The cloning of gp120 from variant BH10 was accomplished by introducing a termination codon at the end of the gp120 gene (amino acid number 511) in plasmid pAcHT3. Details of the cloning strategy are as follows:

Design of oligonucleotide

A synthetic DNA oligonucleotide was designed to be homologous to HIV envelope codons 504 through 510 and pick up homology again 302 bases downstream from codon 510. The nucleotides immediately starting the recurrence of homology between the gp160 gene and the oligonucleotide are TGA, resulting in a stop codon in the frame after the last codon of gp120. A unique restriction enzyme site, BsmI, occurs within the 302-bp region that is important in later steps which allows a powerful selection for the desired mutant. The sequence of the oligonucleotide synthesized by a computerized DNA synthesizer for this experiment is as follows:

5' GAGATTTATTACTCCAACTATCTTTTTTCTCTCTGCACCA 3'

Purification and kinasing of oligonucleotide

Once synthesis cycle was complete, the oligo was deblocked with an equal volume of ammonium hydroxide at 55°C overnight and speed-vacuumed to dryness. The oligo was resuspended in 2 ml dH2O, speed-vacuumed to dryness, and once again resuspended in water and speed-vacuumed to dryness. The final pellet was resuspended in 100 μl dH2O and 1/5th of the total was purified on an 8% acrylamide/urea gel with bromphenol blue (BPB) and xylene cyanole FF (XCFF) dyes as markers. The gel was run at 70 watts until BFB was 3/4 of the way down. The oligonucleotide band was visualized with TLC plates and short-wave U.V. Elution of the oligo from the gel was accomplished by cutting the gel slice into very small pieces and adding two volumes of 0.5 M ammonium acetate, 1 mM EDTA pH 8.0 and incubating the tube at 37°C overnight with shaking. The oligonucleotide was precipitated with ethanol (ETOH) and analyzed by absorbance at wavelength 260 nm. 200 pmole of oligo was kinased in a 40 μl reaction with 2 units of T4 polynucleotide kinase and 0.05 mM ATP (final concentration).

Digests for preparation of template

Plasmid pEc160 was linearized with XmnI which cleaves within pUC 10 sequences away from the inserted envelope gene. A second digest of pEc160 was carried out with restriction enzymes StuI and BamHI, which each cleave the plasmid once within the gp160 gene. StuI is upstream of the gp120/gp41 cleavage point and BamHI is downstream of this point. The larger of the two fragments, 3766 bp, was purified away from the smaller 1632-bp fragment by standard agarose gel techniques.

Preparation of template

Heteroduplex formation using XmnI linearized pEc160 and the 3766-bp fragment described above would result in a circular DNA molecule with the region of DNA between sites StuI and BamHI exposed as single-stranded DNA. This gap of single-stranded DNA serves as a template for hybridization of an oligo designed to place a stop codon at position amino acid number 511 within the gp160 gene. Details of the mutagenesis experiment are as follows:

I. Formation of heteroduplex

    A. 0.4 µg Xmnl linearized pEc160

0.35 µg Stul/BamHI gel-isolated 3766-bp fragment

0.25 µg kinased oligonucleotide

6 µl 10X polymerase buffer

dH₂0 to 35 µl final volume

    B. Mix above ingredients well and remove 8 µl aliquot. (sample 1).

    C. Heat remaining 27 µl to 100°C for 5 min, 37°C for 20 min 4°C for 20 min and transfer to ice bath. Remove a second 8 µl aliquot (sample 2).

II. Polymerization reaction

    A. To remaining 19 µl, add dNTP's at a final concentration of 0.25 mM each; ATP at a final concentration of 0.25 mM; Klenow polymerase (New England Biolabs, Beverley, MA), 1 µl; T4 ligase (New England Biolabs) 1 µl; and dH₂O to 32 µl final volume. Incubate at 16°C overnight. Remove two 8 µl aliquots (samples 3 and 4) .

    B. Inactivate polymerase and ligase. Digest with restriction enzyme unique to plasmid and present in the 302-bp region that you are trying to delete. These two remaining 8 µl aliquots are samples 5 and 6.

III. Evaluation

    A. Samples 1 and 2 are run on an 0.8% agarose gel. If high molecular weight DNA, heteroduplex, is present then proceed.

    B. Sampes 3 and 5 are run on an 0.8% agarose gel. Xmnl linearized DNA not annealed in heteroduplex should be cleaved into two smaller fragments by Bsml enzyme. Heteroduplex DNA that did not have the mutagenic oligo annealed and has been polymerized across the gap will also be linearized by Bsml enzyme. In theory, only correctly formed heteroduplex molecules that have incorporated the mutagenic oligo will not be substrates for the Bsml enzyme since on this molecule the site will be present in a single-strand loop.

    C. Samples 4 and 6 were used to transform E. coli JM103. Ampicillin-resistant colonies were counted from both transformation plates. The number of colonies from sample 4 should be 5- to 10-fold higher than that from Sample 6. Colonies from Sample 6 are then chosen for amplification and plasmid DNA preparations by standard procedures.

IV. Screening/isolation of pHT5

    A. Restriction enzyme sites were chosen that will clearly show wild-type and mutant DNA within the same cell because replication of heteroduplex molecule consistently occurs prior to any repair of the looped-out segment. To screen for deletion of 302 bases, restriction enzyme sites Stul and HindIII, which border the region deleted, were chosen. The pattern predicted for both wild-type and mutant was evident in mini DNA from single colony isolates. DNA from positive colonies above was used to transform E. coli JM105 and mini DNA isolated by standard procedures. Analysis of the Stul/HindIII digestion patterns confirmed pure wild-type clones and pure mutant clones. The clone resulting from the oligonucleotide mutagenesis described above is designated pHT5.

Example 9--Transfer of gp120 gene into vector appropriate for expression in insect cells

    This pUC clone, pHT5, was digested as described in Example 1, No. 7 and cloned into pAc610 as described in Example 1, Nos. 8, 9, and 10. This plasmid is designated pAcHT5 and is appropriate for expressing gp120 from variant BH10 in cultured insect cells. Transfection of Sf9 insect cells with pAcHT5 was done as described for pAcHT3 and the variant gp160 clones. Subsequent steps involved in isolating the recombinant gp120 virus, infecting and growing cultured insect cells is also as described for pAcHT3. The difference in harvesting the growth for purification of gp120 versus gp160 lies in the fact that the majority of the gp120 protein is released into the culture medium.

Example 10--Purification of gp120

    Purification of gp120 from cultured insect cell medium is accomplished by dialysis and concentration of the medium by standard techniques and, subsequently, by affinity chromatography using a column prepared from serum of individuals with Aids Related Complex (ARC). Further purification was accomplished by running the eluant on a 7.5% SDS-polyacrylamid gel as described in Example 5, Nos. 7, 8, and 9 (note: migration was at the position corresponding to 120 kD rather than 160 kD). This material is immunoreactive to antisera raised to gp160 and should have essentially the same anti-HIV immunological properties in relation to virus neutralization as gp160 from insect cells.

Example 11--Cell fusion inhibition

    The in vitro fusion of HIV-infected cells with T4 positive T cells is measured in the presence and absence of immune serum. This is a well-known assay (Putney, et al. [1986] Science 234:1392-1395).

    Chronically infected cells and uninfected cells (1:15) are mixed and incubated 24 hr. Foci of fused cells (giant cells) are then counted (usually about 60). Dilution of an immune serum, for example, serum to the entire HIV envelope (gp160) is added when the cells are mixed. A 90% decrease in giant cells after 24 hr indicates the immune serum can block fusion. This assay can be done with cells infected with various virus strains. Examples for $HIV_{IIIB}$, $HIV_{RF}$, and $HIV_{IIIB/RF}$ hybrid are shown in Table 6.

Results from antisera of two goats immunized with gp160 obtained from the infection of insect cells with the recombinant baculovirus HT3 are shown in Table 5. Controls with pre-immune serum from both goats (average of both is shown) give expected numbers of giant cells from mid 60's to low 70's in contrast with the immune serum values at 1/10 and 1/30 dilutions.

When compared with human serum from an ARC patient at a 1/30 dilution the goat antisera to recombinant gp160 was more effective at blocking cell fusion. When gp120 isolated from $HIV_{IIIB}$ virus was used as an antigen for immunizing goats, the ability to inhibit cell fusion in vitro was not apparent after 48 hr. There was some evidence of slight inhibition at the earlier time of 12 hr.

Examples of cell fusion assays for $HIV_{RF}$ and $HIV_{IIIB/RF}$ hybrid gp160 are shown in Table 6. $HIV_{RF}$ gp160 from clone pAcHT7 when injected into goats also raises antiserum capable of blocking viral-induced cell fusion (goat 1013). Serum from the co-immunization of goat 1014 with gp160 from $HIV_{RF}$ and $HIV_{IIIB}$ clones can efficiently block cell fusion induced by both variants $HIV_{IIIB}$ and $HIV_{RF}$. This result supports the approach for a multivalent vaccine. Interestingly goat 1021 immunized with $HIV_{IIIB/RF}$ hybrid gp160 from clone pAcHT6 shows strong activity against $HIV_{RF}$ and a weaker but still significant activity against $HIV_{IIIB}$ infected cell fusion. This supports the idea that the creation of hybrid gp160 genes can bring about novel immunological properties and that important epitopes can be transferred between HIV variants.

In vitro neutralization assays where the ability of the antisera to neutralize the ability of HIV to infect cells is measured by a reduction in reverse transcriptase levels have also been done. Results from these assays correlate with the results from the cell fusion inhibition assays.

## Table 1
### Amino Acid Sequence of HIV Protein HT7

MetArgValLysGluLysTyrGlnHisLeuTrpArgTrpGlyTrpArgTrpGly

ThrMetLeuLeuGlyMetLeuMetIleCysSerAlaThrGluLysLeuTrpVal

ThrValTyrTyrGlyValProValTrpLysGluAlaThrThrThrLeuPheCys

AlaSerAspAlaLysAlaTyrAspThrGluValHisAsnValTrpAlaThrHis

AlaCysValProThrAspProAsnProGlnGluValValLeuValAsnValThr

GluAsnPheAsnMetTrpLysAsnAspMetValGluGlnMetHisGluAspIle

IleSerLeuTrpAspGlnSerLeuLysProCysValLysLeuThrProLeuCys

ValThrLeuAsnCysThrAspAlaAsnLeuAsnGlyThrAsnValThrSerSer

SerGlyGlyThrMetMetGluAsnGlyGluIleLysAsnCysSerPheGlnVal

ThrThrSerArgArgAspLysThrGlnLysLysTyrAlaLeuPheTyrLysLeu

AspValValProIleGluLysGlyAsnIleSerProLysAsnAsnThrSerAsn

AsnThrSerTyrGlyAsnTyrThrLeuIleHisCysAsnSerSerValIleThr

GlnAlaCysProLysValSerPheGluProIleProIleHisTyrCysThrPro

AlaGlyPheAlaIleLeuLysCysAsnAsnLysLysPheAsnGlyThrGlyPro

CysLysAsnValSerThrValGlnCysThrHisGlyIleArgProValValSer

ThrGlnLeuLeuLeuAsnGlySerLeuAlaGluGluGluValValIleArgSer

GluAsnPheThrAspAsnValLysThrIleIleValGlnLeuAsnAlaSerVal

GlnIleAsnCysThrArgProAsnAsnAsnThrArgLysSerIleThrLysGly

ProGlyArgValIleTyrAlaThrGlyGlnIleIleGlyAspIleArgLysAla

HisCysAsnLeuSerArgAlaGlnTrpAsnAsnThrLeuLysGlnValValThr

LysLeuArgGluGlnPheAspAsnLysThrIleValPheThrSerSerSerGly

GlyAspProGluIleValLeuHisSerPheAsnCysGlyGlyGluPhePheTyr

13

Table 1 (cont.)

CysAsnThrThrGlnLeuPheAsnSerThrTrpAsnSerThrGluGlySerAsn

AsnThrGlyGlyAsnAspThrIleThrLeuProCysArgIleLysGlnIleVal

AsnMetTrpGlnGluValGlyLysAlaMetTyrAlaProProIleSerGlyGln

IleLysCysIleSerAsnIleThrGlyLeuLeuLeuThrArgAspGlyGlyGlu

AspThrThrAsnThrThrGluIlePheArgLeuGlyGlyGlyAsnMetArgAsp

AsnTrpArgSerGluLeuTyrLysTyrLysValValArgIleGluProLeuGly

ValAlaProThrArgAlaLysArgArgValValGlnArgGluLysArgAlaVal

GlyAlaIleGlyAlaMetPheLeuGlyPheLeuGlyAlaAlaGlySerThrMet

GlyAlaGlySerIleThrLeuThrValGlnAlaArgHisLeuLeuSerGlyIle

ValGlnGlnGlnAsnAsnLeuLeuArgAlaIleGluAlaGlnGlnHisLeuLeu

GlnLeuThrValTrpGlyIleLysGlnLeuGlnAlaArgValLeuAlaValGlu

ArgTyrLeuArgAspGlnGlnLeuLeuGlyIleTrpGlyCysSerGlyLysLeu

IleCysThrThrThrValProTrpAsnAlaSerTrpSerAsnLysSerLeuAsn

MetIleTrpAsnAsnMetThrTrpMetGlnTrpGluArgGluIleAspAsnTyr

ThrGlyIleIleTyrAsnLeuLeuGluGluSerGlnAsnGlnGlnGluLysAsn

GluGlnGluLeuLeuGluLeuAspLysTrpAlaAsnLeuTrpAsnTrpPheAsp

IleThrGlnTrpLeuTrpTyrIleArgIlePheIleMetIleValGlyGlyLeu

ValGlyLeuLysIleValPheAlaValLeuSerIleValAsnArgValArgGln

GlyTyrSerProLeuSerPheGlnThrHisLeuProAlaProArgGlyProAsp

ArgProGluGlyIleGluGluGluGlyGlyGluArgAspArgAspArgSerIle

ArgLeuValAsnGlySerLeuAlaLeuIleTrpAspAspLeuArgSerLeuCys

LeuPheSerTyrHisArgLeuArgAspLeuLeuLeuIleValThrArgIleVal

GluLeuLeuGlyArgArgGlyTrpGluAlaLeuLysTyrTrpTrpAsnLeuLeu

GlnTyrTrpSerGlnGluLeuLysAsnSerAlaValSerLeuLeuAsnAlaThr

AlaIleAlaValAlaGluGlyThrAspArgValIleGluValValGlnGlyAla

TyrArgAlaIleArgHisIleProArgArgIleArgGlnGlyLeuGluArgIle

LeuLeu

Table 2

Nucleotide Sequence Encoding HIV Protein HT7

ATGAGAGTGAAGGAGAAATATCAGCACTTGTGGAGATGGGGGTGGAGATGGGGC

ACCATGCTCCTTGGGATGTTGATGATCTGTAGTGCTACAGAAAAATTGTGGGTC

ACAGTCTATTATGGGGTACCTGTGTGGAAGGAAGCAACCACCACTCTATTTTGT

GCATCAGATGCTAAAGCATATGATACAGAGGTACATAATGTTTGGGCCACACAT

GCCTGTGTACCCACAGACCCCAACCCACAAGAAGTAGTATTGGTAAATGTGACA

GAAAATTTTAACATGTGGAAAAATGACATGGTAGAACAGATGCATGAGGATATA

ATCAGTTTATGGGATCAAAGCCTAAAGCCATGTGTAAAATTAACCCCACTCTGT

GTTACTTTAAATTGCACTGATGCTAACTTGAATGGTACTAATGTCACTAGTAGT

AGCGGGGGAACAATGATGGAGAACGGAGAAATAAAAAACTGCTCTTTCCAAGTT

ACCACAAGTAGAAGAGATAAGACGCAGAAAAAATATGCACTTTTTTATAAACTT

GATGTGGTACCAATAGAGAAGGGTAATATTAGCCCTAAGAATAATACTAGCAAT

AATACTAGCTATGGTAACTATACATTGATACATTGTAATTCCTCAGTCATTACA

CAGGCCTGTCCAAAGGTATCCTTTGAGCCAATTCCCATACATTATTGCACCCCG

GCTGGTTTTGCGATTCTAAAGTGTAATAATAAGAAGTTCAATGGAACAGGACCA

TGTAAAAATGTCAGCACAGTACAATGTACACATGGAATTAGGCCAGTAGTGTCA

ACTCAACTGCTGTTAAATGGCAGTCTAGCAGAAGAAGAGGTAGTAATTAGATCT

GAAAATTTCACGGACAATGTTAAAACCATAATAGTACAGCTGAATGCATCTGTA

CAAATTAATTGTACAAGACCCAACAACAATACAAGAAAAAGTATAACTAAGGGA

CCAGGGAGAGTAATTTATGCAACAGGACAAATAATAGGAGATATAAGAAAAGCA

CATTGTAACCTTAGTAGAGCACAATGGAATAACACTTTAAAACAGGTAGTTACA

AAATTAAGAGAACAATTTGACAATAAAACAATAGTCTTTACGTCATCCTCAGGA

GGGGACCCAGAAATTGTACTTCACAGTTTTAATTGTGGAGGGGAATTTTTCTAC

15

Table 2 (cont.)

TGTAATACAACACAACTGTTTAATAGTACTTGGAATAGTACTGAAGGGTCAAAT

AACACTGGAGGAAATGACACAATCACACTCCCATGCAGAATAAAACAAATTGTA

AACATGTGGCAGGAAGTAGGAAAAGCAATGTATGCCCCTCCCATCAGTGGACAA

ATTAAATGTATATCAAATATTACAGGGCTACTATTAACAAGAGATGGGGGTGAA

GATACAACTAATACTACAGAGATCTTCAGACTTGGAGGAGGAAATATGAGGGAC

AATTGGAGAAGTGAATTATATAAATATAAAGTGGTAAGAATTGAGCCATTAGGA

GTGGCACCCACTAGGGCAAAGAGAAGAGTGGTGCAAAGAGAAAAAGAGCAGTG

GGAGCAATAGGAGCTATGTTCCTTGGGTTCTTGGGAGCAGCAGGAAGCACTATG

GGCGCAGGCTCAATAACGCTAACGGTACAGGCCAGACACTTATTGTCTGGTATA

GTGCAACAGCAAAACAATTTGCTGAGGGCTATTGAGGCGCAACAGCATCTGTTG

CAACTCACGGTCTGGGGCATCAAACAGCTCCAGGCAAGAGTCCTGGCTGTGGAA

AGATACCTAAGGGATCAACAGCTCCTAGGAATTTGGGGATGCTCTGGAAAACTC

ATTTGCACCACTACTGTGCCTTGGAATGCTAGTTGGAGTAATAAATCTCTGAAT

ATGATTTGGAATAACATGACCTGGATGCAGTGGGAAAGAGAAATTGACAATTAC

ACAGGCATAATATACAACTTACTTGAAGAATCGCAGAACCAGCAAGAAAAGAAT

GAACAAGAATTATTGGAATTGGATAAATGGGCAAATTTGTGGAATTGGTTTGAC

ATAACACAATGGCTGTGGTATATAAGAATATTCATAATGATAGTAGGAGGCTTG

GTAGGTCTAAAAATAGTTTTTGCTGTGCTTTCTATAGTGAATAGAGTTAGGCAG

GGATACTCACCATTATCATTTCAGACCCACCTCCCAGCCCCGAGGGGACCCGAC

AGGCCCGAAGGAATAGAAGAAGAAGGTGGAGAGAGAGACAGAGACAGATCCATT

CGATTAGTGAACGGATCCTTAGCACTTATCTGGGACGATCTGCGGAGCCTGTGC

CTCTTCAGCTACCACCGCTTGAGAGACTTACTCTTGATTGTAACGAGGATTGTG

GAACTTCTGGGACGCAGGGGGTGGGAAGCCCTCAAATATTGGTGGAATCTCCTA

CAGTATTGGAGTCAGGAGCTAAAGAATAGTGCTGTTAGCTTGCTCAATGCCACA

GCTATAGCAGTAGCTGAGGGGACAGATAGGGTTATAGAAGTAGTACAAGGAGCT

TATAGAGCTATTCGCCACATACCTAGAAGAATAAGACAGGGCTTGGAAAGGATT

TTGCTA

## Table 3
### Amino Acid Sequence of HIV Protein HT6

MetArgValLysGluLysTyrGlnHisLeuTrpArgTrpGlyTrpArgTrpGly

ThrMetLeuLeuGlyMetLeuMetIleCysSerAlaThrGluLysLeuTrpVal

ThrValTyrTyrGlyValProValTrpLysGluAlaThrThrThrLeuPheCys

AlaSerAspAlaLysAlaTyrAspThrGluValHisAsnValTrpAlaThrHis

AlaCysValProThrAspProAsnProGlnGluValValLeuValAsnValThr

GluAsnPheAsnMetTrpLysAsnAspMetValGluGlnMetHisGluAspIle

IleSerLeuTrpAspGlnSerLeuLysProCysValLysLeuThrProLeuCys

ValSerLeuLysCysThrAspLeuLysAsnAspThrAsnThrAsnSerSerSer

GlyArgMetIleMetGluLysGlyGluIleLysAsnCysSerPheAsnIleSer

ThrSerIleArgGlyLysValGlnLysGluTyrAlaPhePheTyrLysLeuAsp

IleIleProIleAspAsnAspThrThrSerTyrThrLeuThrSerCysAsnThr

SerValIleThrGlnAlaCysProLysValSerPheGluProIleProIleHis

TyrCysAlaProAlaGlyPheAlaIleLeuLysCysAsnAsnLysThrPheAsn

GlyThrGlyProCysThrAsnValSerThrValGlnCysThrHisGlyIleArg

ProValValSerThrGlnLeuLeuLeuAsnGlySerLeuAlaGluGluGluVal

ValIleArgSerGluAsnPheThrAspAsnValLysThrIleIleValGlnLeu

AsnAlaSerValGlnIleAsnCysThrArgProAsnAsnAsnThrArgLysSer

IleThrLysGlyProGlyArgValIleTyrAlaThrGlyGlnIleIleGlyAsp

IleArgLysAlaHisCysAsnLeuSerArgAlaGlnTrpAsnAsnThrLeuLys

GlnValValThrLysLeuArgGluGlnPheAspAsnLysThrIleValPheThr

SerSerSerGlyGlyAspProGluIleValLeuHisSerPheAsnCysGlyGly

GluPhePheTyrCysAsnThrThrGlnLeuPheAsnSerThrTrpAsnSerThr

GluGlySerAsnAsnThrGlyGlyAsnAspThrIleThrLeuProCysArgIle

## Table 3 (cont.)

LysGlnIleValAsnMetTrpGlnGluValGlyLysAlaMetTyrAlaProPro

IleSerGlyGlnIleLysCysIleSerAsnIleThrGlyLeuLeuLeuThrArg

AspGlyGlyGluAspThrThrAsnThrThrGluIlePheArgProGlyGlyGly

AspMetArgAspAsnTrpArgSerGluLeuTyrLysTyrLysValValLysIle

GluProLeuGlyValAlaProThrLysAlaLysArgArgValValGlnArgGlu

LysArgAlaValGlyIleGlyAlaLeuPheLeuGlyPheLeuGlyAlaAlaGly

SerThrMetGlyAlaAlaSerMetThrLeuThrValGlnAlaArgGlnLeuLeu

SerGlyIleValGlnGlnGlnAsnAsnLeuLeuArgAlaIleGluAlaGlnGln

HisLeuLeuGlnLeuThrValTrpGlyIleLysGlnLeuGlnAlaArgIleLeu

AlaValGluArgTyrLeuLysAspGlnGlnLeuLeuGlyIleTrpGlyCysSer

GlyLysLeuIleCysThrThrAlaValProTrpAsnAlaSerTrpSerAsnLys

SerLeuGluGlnIleTrpAsnAsnMetThrTrpMetGluTrpAspArgGluIle

AsnAsnTyrThrSerLeuIleHisSerLeuIleGluGluSerGlnAsnGlnGln

GluLysAsnGluGlnGluLeuLeuGluLeuAspLysTrpAlaSerLeuTrpAsn

TrpPheAsnIleThrAsnTrpLeuTrpTyrIleLysLeuPheIleMetIleVal

GlyGlyLeuValGlyLeuArgIleValPheAlaValLeuSerValValAsnArg

ValArgGlnGlyTyrSerProLeuSerPheGlnThrHisLeuProIleProArg

GlyProAspArgProGluGlyIleGluGluGluGlyGlyGluArgAspArgAsp

ArgSerIleArgLeuValAsnGlySerLeuAlaLeuIleTrpAspAspLeuArg

SerLeuCysLeuPheSerTyrHisArgLeuArgAspLeuLeuLeuIleValThr

ArgIleValGluLeuLeuGlyArgArgGlyTrpGluAlaLeuLysTyrTrpTrp

AsnLeuLeuGlnTyrTrpSerGlnGluLeuLysAsnSerAlaValSerLeuLeu

AsnAlaThrAlaIleAlaValAlaGluGlyThrAspArgValIleGluValVal

GlnGlyAlaTyrArgAlaIleArgHisIleProArgArgIleArgGlnGlyLeu

GluArgIleLeuLeu

## Table 4
### Nucleotide Sequence Encoding HIV Protein HT6

```
ATGAGAGTGAAGGAGAAATATCAGCACTTGTGGAGATGGGGGTGGAGATGGGGC

ACCATGCTCCTTGGGATGTTGATGATCTGTAGTGCTACAGAAAAATTGTGGGTC

ACAGTCTATTATGGGGTACCTGTGTGGAAGGAAGCAACCACCACTCTATTTTGT

GCATCAGATGCTAAAGCATATGATACAGAGGTACATAATGTTTGGGCCACACAT

GCCTGTGTACCCACAGACCCCAACCCACAAGAAGTAGTATTGGTAAATGTGACA

GAAAATTTTAACATGTGGAAAAATGACATGGTAGAACAGATGCATGAGGATATA

ATCAGTTTATGGGATCAAAGCCTAAAGCCATGTGTAAAATTAACCCCACTCTGT

GTTAGTTTAAAGTGCACTGATTTGAAGAATGATACTAATACCAATAGTAGTAGC

GGGAGAATGATAATGGAGAAAGGAGAGATAAAAAACTGCTCTTTCAATATCAGC

ACAAGCATAAGAGGTAAGGTGCAGAAAGAATATGCATTTTTTTATAAACTTGAT

ATAATACCAATAGATAATGATACTACCAGCTATACGTTGACAAGTTGTAACACC

TCAGTCATTACACAGGCCTGTCCAAAGGTATCCTTTGAGCCAATTCCCATACAT

TATTGTGCCCCGGCTGGTTTTGCGATTCTAAAATGTAATAATAAGACGTTCAAT

GGAACAGGACCATGTACAAATGTCAGCACAGTACAATGTACACATGGAATTAGG

CCAGTAGTATCAACTCAACTGCTGTTAAATGGCAGTCTGGCAGAAGAAGAGGTA

GTAATTAGATCTGAAAATTTCACGGACAATGTTAAAACCATAATAGTACAGCTG

AATGCATCTGTACAAATTAATTGTACAAGACCCAACAACAATACAAGAAAAAGT

ATAACTAAGGGACCAGGGAGAGTAATTTATGCAACAGGACAAATAATAGGAGAT

ATAAGAAAAGCACATTGTAACCTTAGTAGAGCACAATGGAATAACACTTTAAAA

CAGGTAGTTACAAAATTAAGAGAACAATTTGACAATAAAACAATAGTCTTTACG

TCATCCTCAGGAGGGGACCCAGAAATTGTACTTCACAGTTTTAATTGTGGAGGG

GAATTTTTCTACTGTAATACAACACAACTGTTTAATAGTACTTGGAATAGTACT

GAAGGGTCAAATAACACTGGAGGAAATGACACAATCACACTCCCATGCAGAATA

AAACAAATTGTAAACATGTGGCAGGAAGTAGGAAAAGCAATGTATGCCCCTCCC
```

## Table 4 (cont.)

```
ATCAGTGGACAAATTAAATGTATATCAAATATTACAGGGCTACTATTAACAAGA
GATGGGGGTGAAGATACAACTAATACTACAGAGATCTTCAGACCTGGAGGAGGA
GATATGAGGGACAATTGGAGAAGTGAATTATATAAATATAAAGTAGTAAAAATT
GAACCATTAGGAGTAGCACCCACCAAGGCAAAGAGAAGAGTGGTGCAGAGAGAA
AAAAGAGCAGTGGGAATAGGAGCTTTGTTCCTTGGGTTCTTGGGAGCAGCAGGA
AGCACTATGGGCGCAGCGTCAATGACGCTGACGGTACAGGCCAGACAATTATTG
TCTGGTATAGTGCAGCAGCAGAACAATTTGCTGAGGGCTATTGAGGCGCAACAG
CATCTGTTGCAACTCACAGTCTGGGGCATCAAGCAGCTCCAGGCAAGAATCCTG
GCTGTGGAAAGATACCTAAAGGATCAACAGCTCCTGGGGATTTGGGGTTGCTCT
GGAAAACTCATTTGCACCACTGCTGTGCCTTGGAATGCTAGTTGGAGTAATAAA
TCTCTGGAACAGATTTGGAATAACATGACCTGGATGGAGTGGGACAGAGAAATT
AACAATTACACAAGCTTAATACACTCCTTAATTGAAGAATCGCAAAACCAGCAA
GAAAAGAATGAACAAGAATTATTGGAATTAGATAAATGGGCAAGTTTGTGGAAT
TGGTTTAACATAACAAATTGGCTGTGGTATATAAAATTATTCATAATGATAGTA
GGAGGCTTGGTAGGTTTAAGAATAGTTTTTGCTGTACTTTCTGTAGTGAATAGA
GTTAGGCAGGGATATTCACCATTATCGTTTCAGACCCACCTCCCAATCCCGAGG
GGACCCGACAGGCCCGAAGGAATAGAAGAAGAAGGTGGAGAGAGAGACAGAGAC
AGATCCATTCGATTAGTGAACGGATCCTTAGCACTTATCTGGGACGATCTGCGG
AGCCTGTGCCTCTTCAGCTACCACCGCTTGAGAGACTTACTCTTGATTGTAACG
AGGATTGTGGAACTTCTGGGACGCAGGGGGTGGGAAGCCCTCAAATATTGGTGG
AATCTCCTACAGTATTGGAGTCAGGAGCTAAAGAATAGTGCTGTTAGCTTGCTC
AATGCCACAGCTATAGCAGTAGCTGAGGGGACAGATAGGGTTATAGAAGTAGTA
CAAGGAGCTTATAGAGCTATTCGCCACATACCTAGAAGAATAAGACAGGGCTTG
GAAAGGATTTTGCTA
```

Table 5

ACTIVITY OF SERIAL BLEEDS FROM TWO GOATS IMMUNIZED WITH r160

| Antiserum | Bleed Date (Post immun.) wks. | Cell Fusion Assay Average Number of Giant Cells at Serum Dilution | | |
|---|---|---|---|---|
| | | 1/10 | 1/30 | 1/90 |
| Goat Anti r160 993 | 6 | 0 | 0 | 47 |
| | 9 | 0 | 5 | 48 |
| Goat Anti r160 994 | 6 | 0 | 7 | 49 |
| | 9 | 0 | 1 | 48 |
| Preimmune Goat | | 63 | 72 | 64 |
| Human | | 0 | 42 | 61 |
| gp120 | | 57 | 55 | 60 |

Chronically infected CEM cells (HIV$_{IIIB}$), uninfected CEM cells and the indicated dilution of sera were incubated at 37$^{o}$. The number of giant cell foci were determined after 48 hours.

Table 6

## CELL FUSION ASSAYS

| Goat | rgp160 Immunogen(s) | Bleed Date Post Immun weeks | | $HIV_{IIIB}$ | $HIV_{MN}$ | $HIV_{RF}$ |
|---|---|---|---|---|---|---|
| 1013 | $HIV_{RF}$ | lst | 6 | 38 | 41 | 0 |
| | | 2nd | 9 | N.T. | N.T. | 0 |
| 1014 | $HIV_{RF}$ +$HIV_{IIIB}$ | lst | 6 | 1 | 51 | 0 |
| | | 2nd | 9 | 2 | N.T. | 0 |
| 1021 | $HIV_{IIIB/RF}$ Hybrid | 1st | 6 | 3 | 41 | 0 |
| | | 2nd | 9 | 24 | N.T. | 3 |
| No Serum | | Assay 1 | | 46 | 40 | 42 |
| | | Assay 2 | | 47 | N.T. | 58 |

N.T. = Not Tested

# 0 272 858

## Claims

1. Insect cells infected with a recombinant Baculovirus containing DNA encoding a polypeptide selected from HIV envelope protein gp160 or gp120, and variants and hybrids thereof.

2. Insect cells according to claim 1, wherein the polypeptide is the HIV envelope protein or a polypeptide analogous thereto in that it gives substantially the same combination of humoral and cellular immunity to an organism.

3. Insect cells according to claim 1 or claim 2, wherein the polypeptide is the HIV envelope protein, variant RF of protein gp160, the hybrid from variants BH10 and RF, or variant BH of protein gp120.

4. Insect cells according to any preceding claim, wherein the recombinant Baculovirus is Autographa californica nuclear polyhedrosis virus.

5. Insect cells according to any preceding claim, wherein the insect is Spodoptera frugiperda.

6. A recombinant Baculovirus selected from HT3, HT6, HT7 and HT5.

7. Insect cells according to any of claims 1 to 5, wherein the Baculovirus is as claimed in claim 6.

8. A process for preparing insect cells according to any of claims 1 to 5 and 7, which comprises:
(a) constructing a DNA transfer vector comprising DNA encoding the polypeptide;
(b) transfecting the vector into insect cells with Baculovirus;
(c) isolating the recombinant Baculovirus encoding the polypeptide; and
(d) infecting insect cells with the recombinant Baculovirus.

9. A process according to claim 8, wherein the DNA transfer vector is a bacterial plasmid.

10. A plasmid selected from pAcHT3, pAcHT6, pAcHT7 and pAcHT5.

11. A process according to claim 9, wherein the plasmid is as claimed in claim 10.

12. A process for preparing a polypeptide as defined in any of claims 1 to 3, which comprises recovering the polypeptide from infected insect cells according to any of claims 1 to 5 and 7 or produced by a process according to any of claims 8, 9 and 11.

13. A progress according to claim 12, which comprises growing the infected insect cells in a suitable medium; and recovering and purifying the polypeptide.

14. A bacterial host transformed by the DNA transfer vector defined in any of claims 8, 9 and 11.

15. Insect cells, e.g. of Spodoptera frugiperda, transfected by the DNA transfer vector defined in any of claims 8, 9 and 11.

16. E. Coli JM103, a host according to claim 14.

17. An immunochemical assay for detecting or quantifying antibody against HIV in a fluid sample, which comprises contacting the sample with a polypeptide as defined in any of claims 1 to 3 or the product of a process according to claim 12 or claim 13.

18. A method according to claim 17, which comprises
(a) incubating an immunoadsorbent comprising a solid phase to which the polypeptide is attached with the sample, under conditions which allow the anti-HIV antibody in the sample to bind to the immunoadsorbent;
(b) separating the immunoadsorbent from the sample; and
(c) determining if antibody has bound to the immunoadsorbent as an indication of anti-HIV in the sample.

19. A method according to claim 18, wherein step (c) comprises incubating the immunoadsorbent with a labelled antibody against antigen of the species from which the biological fluid is derived; separating the immunoadsorbent from the labelled antibody after the incubation; and detecting the label associated with the immunoadsorbent.

20. A method according to claim 19, wherein the antibody of the labelled antibody is protein A or a polypeptide as defined in any of claims 1 to 3.

21. A method acording to claim 17, of detecting antobidy against HIV in a human serum or plasma sample, which comprises:
(a) incubating an immunoadsorbent comprising a bead coated with a polypeptide as defined in any of claims 1 to 3 with the sample, under conditions which allow anti-HIV antibody in the sample to bind to the immunoadsorbent;
(b) separating the immunoadsorbent from the sample;
(c) incubating the immunoadsorbent with a labelled anti-(human IgG) antibody under conditions which allow the anti-(human IgG) antibody to bind human anti-HIV antibody bound to the immunoadsorbent;
(d) separating the immunoadsorbent from the unbound anti-(human IgG) antibody; and
(e) evaluating the label associated with the immunoadsorbent as an indication of the presence of antibody against HIV in the sample.

22. A method according to claim 21, wherein the immunoadsorbent further comprises a post-coat of animal protein.

23. A method according to claim 21 or claim 22, wherein the anti-(human IgG) antibody is an animal

23

0272858

Fig. 1